# EUROPÄISCHE PATENTSCHRIFT

(11) **EP 1 269 190 B1**
(45) Veröffentlichungstag und Bekanntmachung des Hinweises auf die Patenterteilung: **06.05.2004**
(21) Anmeldenummer: 01925304.6
(22) Anmeldetag: 17.03.2001
(51) Int. Cl.: G01N 33/543, G01N 33/538, B01L 3/00

(54) **VERFAHREN ZUM NACHWEISEN UND/ODER QUANTIFIZIEREN ERSTER MOLEKÜLE**
METHOD FOR DETECTING AND/OR QUANTIFYING FIRST MOLECULES
PROCEDE DE DETECTION ET/OU DE QUANTIFICATION DE PREMIERES MOLECULES

(30) Priorität: 29.03.2000 DE 10015448
(43) Veröffentlichungstag der Anmeldung: 02.01.2003
(73) Patentinhaber: November Aktiengesellschaft Gesellschaft für Molekulare Medizin, 91056 Erlangen (DE)
(72) Erfinder: KOSAK, Hans, 53123 Bonn (DE); KRAUSE, Jürgen, 91052 Erlangen (DE)
(74) Vertreter: Gassner, Wolfgang, Dr.
(86) Internationale Anmeldenummer: PCT/DE2001/001078
(87) Internationale Veröffentlichungsnummer: WO 2001/073432

(56) Entgegenhaltungen:
- EP-A- 0 520 202
- EP-A- 0 942 283
- WO-A-99/32886
- DE-A- 19 500 862
- US-A- 5 045 479

## Beschreibung

Die Erfindung betrifft Verfahren zum Nachweisen und/oder Quantifizieren des Bindens erster Moleküle an dazu affine zweite Moleküle. Sie betrifft ferner Verfahren zum Nachweisen und/oder Quantifizieren der enzymatischen oder chemischen Aktivität erster Moleküle zu dazu affinen zweiten oder dritten Molekülen. Die Erfindung betrifft außerdem eine Mikrotiterplatte zur Durchführung des Verfahrens und eine Verwendung.

Bekannt sind Verfahren mit folgenden Schritten:
A) Inkontaktbringen von eine Markierungssubstanz aufweisenden ersten Molekülen mit dazu affinen, immobilisierten zweiten Molekülen in einem Bindungsansatz,
B) Inkubieren des Bindungsansatzes,
C) Detektieren einer Eigenschaft der Markierungssubstanz an denjenigen ersten Molekülen, die an zweite Moleküle gebundenen sind.

Für Verfahren mit hohem Probendurchsatz (High-Throughput-Screening) ist es günstig Schritt lit. C durchzuführen, ohne ungebundene erste Moleküle aus dem Bindungsansatz zu entfernen. Folgende Verfahren ohne Entfernung der ersten Moleküle sind bekannt:
- Detektieren einer Fluoreszenz-Polarisation. Die Markierungssubstanz ist ein Fluorophor. Gemessen wird die Änderung der Fluoreszenz-Polarisation der Markierungssubstanz durch das Binden.
- Detektieren einer durch einen Energie-Transfer bedingten Änderung einer Fluoreszenz. Die Markierungssubstanz ist entweder ein Fluoreszenz-Donor oder ein Fluoreszenz-Akzeptor. Durch das Binden verringert sich der Abstand der ersten Moleküle zu den zweiten Molekülen. Das ermöglicht einen Energie-Transfer zwischen der Markierungssubstanz und einem weiteren, in der Nähe der zweiten Moleküle angeordneten Fluoreszenz-Akzeptor bzw. Fluoreszenz-Donor.
- Detektieren einer Änderung der Verweildauer der ersten Moleküle in einem vorgegebenen Volumen. Bei diesem auch als Fluoreszenz-Korrelations-Spektroskopie bekannten Verfahren ist die Markierungssubstanz ein Fluorophor. Die Geschwindigkeit der Molekularbewegung der ersten Moleküle verringert sich durch die Bindung an die zweiten Moleküle. Die Verweildauer des Fluorophors in dem vorgegebenen Volumen erhöht sich.

Darüber hinaus gibt es Testsysteme, bei denen das Binden unmarkierter erster Moleküle an zweite Moleküle indirekt detektiert wird:
- Die zweiten Moleküle sind an Mikropartikeln immobilisiert. An den zweiten Molekülen sind fluoreszierende dritte Moleküle spezifisch gebunden. Das Binden der ersten Moleküle an die zweiten Moleküle verdrängt die gebundenen dritten Moleküle von den Bindungsstellen. Die dadurch bedingte Änderung der Fluoreszenz der Mikropartikel wird detektiert.
- Die zweiten Moleküle sind an einer Gefäßwand immobilisiert, die einen Szintillator enthält. An die zweiten Moleküle sind dritte Moleküle spezifisch gebunden, die eine radioaktive Markierungssubstanz aufweisen. Die Markierungssubstanz verursacht eine meßbare Szintillation. Das Binden der ersten Moleküle an die zweiten Moleküle verdrängt die gebundenen dritten Moleküle von den Bindungsstellen. Die dadurch bedingte Änderung der Szintillation wird gemessen.

Die bekannten homogenen Testsysteme sind technisch aufwendig und benötigen zum Teil gesundheitsgefährdende Substanzen.

Die WO 95/18376 beschreibt ein Verfahren zur Ermittlung der Konzentration eines Analyten in einer Flüssigkeit. Dabei wird die Flüssigkeit mit einer an einer festen Phase immobilisierten Sonde in Kontakt gebracht, welche den Analyten spezifisch bindet. Anschließend wird der Anteil der belegten und der nicht belegten Bindungsstellen der Sonde mittels Rücktitration bestimmt. Das Ergebnis wird mit einem zuvor ermittelten Standart verglichen. - Das verwendete Verfahren erfordert viele Schritte. Es ist zeitaufwendig und anfällig gegenüber Verunreinigungen.

Die US 6,020,207 offenbart ein Verfahren zur optischen Detektion chemischer Verbindungen. Dabei ist eine Sonde an der Innenwand einer Kapillare immobilisiert. Eine den Analyten enthaltende Lösung wird in die Kapillare gefüllt. Zur Detektion der chemischen Verbindung wird die durch die Bindung der chemischen Verbindung an die Sonde bewirkte geänderte Lichtabsorption an der Innenwand der Kapillare gemessen. - Die Messung ist kompliziert und erfordert einen hohen apparativen Aufwand.

Die WO 99/44065 beschreibt ein Verfahren zur Detektion eines Analyten in einer Probe. Dabei wird die den Analyt enthaltene Lösung durch eine Kapillare gepumpt, an deren Innenwand eine Sonde immobilisiert ist. An die Sonde sind Verbindungen gebunden, welche eine mittels modifizierter Raman-Spektroskopie erfaßbare Markierung aufweisen. Bei Vorliegen des Analyten werden die Verbindungen freigesetzt und können mittels modifizierter Raman-Spektroskopie nachgewiesen werden.

Die DE 44 07 142 A1 betrifft einen heterologen Immonoassay, bei dem eine Sonde auf einem festen Träger, z.B. einer Durchflußküvette, immobilisiert ist. Ein in einer Lösung enthaltener Analyt wird an die Sonde gebunden, anschließend wieder gelöst und z.B. mittels Fluoreszenz oder UV-Absorption nachgewiesen.

Die DE 198 28 837 A1 beschreibt ein Verfahren, bei dem eine Sonde an einem Ring immobilisiert ist. Zum Nachweis von an die Sonde gebundenem Analyt kann der Ring in eine Reaktionskammer eines UV-Analysengeräts eingespannt werden. Das vorgeschlagene Nachweisverfahren ist aufwendig.

Aufgabe der vorliegenden Erfindung ist es, die Nachteile des Stands der Technik zu beseitigen. Insbesondere sollen Verfahren, eine Mikrotiterplatte und eine Verwendung bereitgestellt werden, mit denen einfach und kostengünstig ein hoher Probendurchsatz erzielbar ist. Nach dem Inkontaktbringen der zweiten Moleküle mit den ersten Molekülen soll kein weiterer Pipettier- oder Waschschritt erforderlich sein.

Diese Aufgabe wird durch die Merkmale der Ansprüche 1 bis 5, 34 und 40 gelöst. Zweckmäßige Ausgestaltungen ergeben sich aus den Merkmalen der Ansprüche 6 bis 33, 35 bis 39 sowie 41 und 42.

Das erfindungsgemäße Verfahren eignet sich zum Nachweis und/oder Quantifizieren des Bindens erster Moleküle an dazu affine zweite Moleküle. Es eignet sich ebenso zum Quantifizieren der enzymatischen oder chemischen Aktivität erster Molekülen zu dazu affinen zweiten oder dritten Molekülen. Die Verfahrensschritte lit. a bis d ermöglichen jeweils eine einfache, schnelle und kostengünstige Verfahrensführung.

Das Detektieren der Konzentration beim Schritt lit. d kann mittels elektromagnetischer Strahlung, wie Licht, oder mittels Teilchenstrahlung, wie Neutronenstrahlung, erfolgen. Es findet im Gefäß vorzugsweise in einem Bereich statt, der möglichst weit von der Wand entfernt ist. Das erhöht die Genauigkeit des Verfahrens. Die erfindungsgemäßen Verfahren haben den Vorteil, mit einem geringen Pipettieraufwand durchführbar zu sein. Sie sind einfach zu handhaben und kostengünstig. Sie erfordern einen geringen Bedarf an Material und Raum. Sie ermöglichen einen hohen Probendurchsatz und sind gut zur Automatisierung geeignet. Das wird insbesondere dadurch erreicht, daß nach dem Inkontaktbringen der zweiten Moleküle mit einer die ersten Moleküle enthaltenden Lösung keine weiteren mechanischen Arbeitsschritte, insbesondere keine Trenn- und Waschschritte, erforderlich sind. Durch die geringe Zahl der Arbeitsschritte ist die Gefahr der Verfälschung der Ergebnisse, insbesondere durch Pipettierfehler, gering. Das Verfahren kann gleichzeitig oder in kurzer Abfolge mit einer großen Zahl von Gefäßen und unterschiedlichen ersten, zweiten, dritten und/oder vierten Molekülen durchgeführt werden. Das Verfahren ist in idealer Weise für die Suche nach neuen pharmakologischen Wirkstoffen geeignet.

Bei den Verfahren zum Nachweisen und/oder Quantifizieren des Bindens (= Verfahrensgruppe I) unter Verwendung dritter oder vierter Molekülen ist es besonders vorteilhaft, wenn diese mit den zweiten Molekülen oder der Wand assoziiert sind. Auf diese Art und Weise ist es nicht erforderlich die dritten oder vierten Moleküle in einem Pipettierschritt der Lösung oder dem Gefäß hinzuzufügen.

Weiterhin ist es bei den Verfahren gemäß Verfahrensgruppe I mit vierten Molekülen vorteilhaft, wenn die Affinität der vierten Moleküle zu den zweiten Molekülen nicht oder nicht wesentlich größer ist als die Affinität der ersten Moleküle zu den zweiten Molekülen. Das erleichtert das Verdrängen der vierten Moleküle von deren Bindungsstellen an den zweiten Molekülen. Auch die Bindung der vierten Moleküle an die zweiten Moleküle wird leichter verhindert.

Vorteilhafterweise ist bei den Verfahren gemäß Verfahrensgruppe I mit vierten Molekülen die Anzahl der ersten Moleküle größer als die Anzahl der zweiten Moleküle und die Anzahl der zweiten Moleküle ist größer als die Anzahl der vierten Moleküle. Das bewirkt eine Kompetition der ersten Moleküle und der vierten Moleküle um die Bindungsstellen an den zweiten Molekülen. Die Konzentration der vierten Moleküle wird stark durch die Konzentration der ersten Moleküle und deren Affinität zu den zweiten Molekülen beeinflußt.

Bei einem Verfahren der Verfahrensgruppe I ohne vierte Moleküle ist es bevorzugt, daß die Anzahl der zweiten Moleküle größer ist als die Anzahl der ersten Moleküle. Das führt zu einer schnellen und deutlichen Konzentrationsänderung der ersten Moleküle oder der ersten und dritten Moleküle in der im Gefäß enthaltenen Lösung. Bei einem Verfahren mit dritten Molekülen ist es vorteilhaft, wenn die Anzahl der ersten Moleküle nicht kleiner ist als die Anzahl der dritten Moleküle. Das verhindert, daß nach der Bindung der ersten Moleküle an die zweiten Moleküle in der Lösung dritte Moleküle verbleiben. In der Lösung verbleibende dritte Moleküle bewirken bei Schritt lit. d ein Hintergrundsignal.

Bei den Verfahren zum Nachweis und/oder Quantifizierung der enzymatischen oder chemischen Aktivität erster Moleküle (= Verfahrensgruppe II) kann das erste Molekül ein spaltendes Enzym, vorzugsweise eine Protease, eine Peptidase, Nuklease, Helicase oder Lipase, oder ein Zucker-abbauendes Enzym sein. Das zweite Molekül kann ein, vorzugsweise aus einem Protein, Peptid, einer Nukleinsäure, Helicase oder einem monomeren oder polymeren Zucker gebildetes, Substrat für das erste Molekül bilden. Es ist ferner möglich, daß das dritte Molekül ein spaltendes Enzym, vorzugsweise eine Protease, Peptidase, Nuklease, Helicase oder Lipase oder ein Zucker-abbauendes Enzym ist. Das zweite Molekül bildet zweckmäßigerweise ein, vorzugsweise aus einem Protein, Peptid, einer Nukleinsäure, Helicase oder einem monomeren oder polymeren Zucker gebildetes, Substrat für das dritte Molekül. Das erste Molekül kann in Bezug zum dritten Molekül ein Agonist, ein Antagonist oder ein Kompetitor sein. Ferner ist es zweckmäßig, daß durch die Bindung des zweiten Moleküls an das erste Molekül eine Spaltung des zweiten Moleküls durch das dritte Molekül verhindert wird.

Bei einer vorteilhaften auf beide Verfahrensgruppen gleichermaßen anwendbaren Ausgestaltung wird zum Detektieren mit einem Lichtstrahl, insbesondere einer definierten Wellenlänge, vorzugsweise einem Laserstrahl, in die Lösung eingestrahlt. Der Lichtstrahl kann parallel zu der Wand eingestrahlt werden. Er kann polarisiert sein. Zum Detektieren kann eine Fluoreszenz, eine Streuung eine Absorption oder eine optische Aktivität gemessen werden.

Vorteilhafterweise enthält die Lösung zusätzlich fünfte, als interne Marker dienende Moleküle, die keine spezifische Affinität zu den ersten, zweiten, dritten oder vierten Molekülen aufweisen. Die fünften Moleküle können dazu eingesetzt werden, eine Volumen- und damit eine Konzentrationsänderung durch Verdunsten von Wasser festzustellen. Weiterhin können die fünften Moleküle dazu dienen, das Maß unspezifischer Bindungen zu ermitteln. Dabei zeigt eine Abnahme der Konzentration der fünften Moleküle eine unspezifische Bindung an die Wand und/oder die zweiten Moleküle an.

In einem bevorzugten Ausführungsbeispiel sind die ersten, dritten, vierten und/oder fünften Moleküle oder damit assoziierende Bestandteile der Lösung fluoreszierend, lichtstreuend, lichtabsorbierend oder optisch aktiv. Damit assoziierte Bestandteile der Lösung können beispielsweise Antikörper sein, welche die ersten, dritten, vierten und/oder fünften Moleküle spezifische binden können.

Vorteilhafterweise erfolgt die Durchführung des Schritts lit. d durch mehrfaches oder kontinuierliches Bestimmen der Konzentration der ersten, dritten oder vierten Moleküle während der Durchführung des Schritts lit. c, insbesondere an deren Beginn und Ende. Das Bestimmen der Konzentration kann beispielsweise durch Vergleichen der optischen Eigenschaften der Lösung mit denjenigen verschiedener Eichlösungen erfolgen.

Bevorzugt sind freie Bindungsstellen an der Wand des Gefäßes durch daran gebundene sechste Moleküle abgesättigt. Die sechsten Moleküle sollten ähnliche physikalisch/chemische Eigenschaften, wie Ladung, Molekülgröße usw., aufweisen, wie die zweiten Moleküle. Sie sollten jedoch keine Affinität zu den ersten Molekülen besitzen. Dadurch kann eine unspezifische Bindung an freie Bindungsstellen an der Wand des Gefäßes unterdrückt werden. Unspezifisch ist jede Bindung, die nicht an den spezifischen Bindungsstellen der zweiten Moleküle erfolgt.

Vorteilhafterweise enthält die Lösung mindestens einen eine unspezifische Bindung inhibierenden Zusatz, insbesondere ein Detergenz, ein Protein, ein Proteingemisch oder ein Salz. Der Zusatz unterdrückt nicht die spezifische Bindung der ersten Moleküle an den spezifischen Bindungsstellen der zweiten Moleküle. Er inhibiert unspezifische Bindungen der ersten, zweiten, dritten, vierten und fünften Moleküle untereinander und an der Wand des Gefäßes bzw. den dort gebundenen sechsten Molekülen. Als Detergenzien kommen z.B. Tween-20, Nonidet oder SDS in Betracht. Als Salze sind solche geeignet, die unspezifische Ionenbindungen unterdrücken. Proteine oder Proteingemische können z.B. Magermilch, Rinderserumalbumin oder Casein sein.

Bei einer vorteilhaften Ausgestaltung wird zur Ermittlung einer spezifischen Konzentrationsänderung von dem Betrag der Konzentrationsänderung gemäß Schritt lit. d der Betrag einer Konzentrationsänderung bei Durchführung des Verfahrens ohne zweite Moleküle abgezogen. Spezifisch ist eine Konzentrationsänderung, die nur durch eine Bindung der ersten Moleküle an die zweiten Moleküle verursacht wird.

Bevorzugt sind die zweiten Moleküle an einer vorgegebenen Stelle ihrer Struktur immobilisiert. Das ermöglicht es, ein Immobilisieren der zweiten Moleküle an derjenigen Stelle ihrer Struktur zu vermeiden, die affin zu den zweiten bzw. vierten Molekülen ist. Ein solches Immobilisieren würde die Zugänglichkeit dieser Stellen für dazu affine erste bzw. vierte Moleküle einschränken. Vorteilhafterweise sind die zweiten Moleküle über Linker oder Spacer an der Wand gebunden. Das verbessert die Zugänglichkeit der zweiten Moleküle. Linker können das Immobilisieren von zweiten Molekülen, die sich direkt nur schlecht oder gar nicht immobilisieren lassen, verbessern oder ermöglichen.

Vorzugsweise ist das Gefäß als Kavität einer Mikrotiterplatte mit mindestens 96, insbesondere 384, Kavitäten ausgebildet. Bei einer bevorzugten Ausgestaltung weist das Gefäß an der Ein- und/oder Austrittstelle des Lichtstrahls, vorzugsweise dem Boden des Gefäßes, im wesentlichen keine immobilisierten zweiten Moleküle auf. Das verhindert beim Schritt lit. d eine Störung des Detektierens durch erste, dritte oder vierte Moleküle, die direkt oder indirekt an immobilisierte zweite Moleküle an der Ein- und/oder Austrittsstelle des Lichtstrahls gebunden sind. Besonders vorteilhaft ist es, wenn das Gefäß eine an den Enden offene Kapillare ist. Unter einer Kapillare wird hier ein an beiden Seiten offenes Röhrchen mit einem Innendurchmesser von bis zu 2 Millimeter verstanden. Die Kapillare kann von dem Lichtstrahl so durchstrahlt werden, daß dieser die Wand der Kapillare nicht bestrahlt. Eine Beeinflussung des Detektierens durch gebundene erste, dritte oder vierte Moleküle ist ausgeschlossen. Vorzugsweise wird die Kapillare mittels Kapillarkräften mit der Lösung gefüllt. Dadurch ist die Aufnahme eines definierten Volumens gewährleistet. Ein Pipettierschritt ist nicht erforderlich. Eine dadurch bedingte Fehlerquelle ist ausgeschlossen.

Bevorzugt ist der Quotient aus der Fläche der Wand in mm² und dem Volumen der Lösung in mm³ größer als 1 mm⁻¹, vorzugsweise größer als 3 mm⁻¹. Das Gefäß ist in diesem Fall länglich. Ein vorgegebenes Volumen kann mit einer großen Wandfläche mit immobilisierten zweiten Molekülen in Kontakt gebracht werden. Gleichzeitig ist ein langer Weg des Lichtstrahls durch die Lösung gewährleistet. Die Empfindlichkeit des Verfahrens kann durch eine längliche Geometrie des Gefäßes erheblich gesteigert werden.

Die ersten, zweiten, dritten, vierten, fünften oder sechsten Moleküle können aus folgender Gruppe ausgewählt sein: Peptide, Proteine, Nukleinsäuren, Zucker, Polymere, Botenstoffe, Zellen, Zellfragmente, Viren, deren Bestandteile oder Fragmente dieser Bestandteile, Kapside, deren Bestandteile oder Fragmente dieser Bestandteile und Hormone. Bei einer vorteilhaften Ausgestaltung der Erfindung wird das erfindungsgemäße Verfahren gleichzeitig oder in kurzer Abfolge an einer Anzahl von Gefäßen, insbesondere Kapillaren, durchgeführt.

Besonders gut zur Durchführung des erfindungsgemäßen Verfahrens geeignet ist eine Mikrotiterplatte mit einer Vielzahl jeweils eine Wand und einen Boden aufweisenden Kavitäten, wobei die Wand für die Bindung der Moleküle aktiviert ist und wobei der Boden nicht für die Bindung zweiter Moleküle aktiviert ist. Eine solche Mikrotiterplatte erlaubt es dem Benutzer, an der Wand jeweils die ihn interessierenden zweiten Moleküle selbst zu immobilisieren.

Die Aktivierung der zweckmäßigerweise aus Kunststoff hergestellten Mikrotiterplatte erfolgt nach herkömmlichen Verfahren. Eine Aktivierung des Bodens der Kavität kann verhindert werden, indem der Boden z.B. während der Aktivierung abgedeckt wird. Es ist aber auch möglich, den Boden als separates Teil auszuführen und an die bereits aktivierten Wände anzufügen.

Nach einem weiteren Ausgestaltungsmerkmal sind an der Wand zweite Moleküle immobilisiert und der Boden weist im wesentlichen keine immobilisierten zweiten Moleküle auf. Dabei können an der Wand jeweils unterschiedliche zweite Moleküle immobilisiert sein. Das erlaubt eine Verfahrensführung mit einem hohen Durchsatz.

Vorteilhafterweise ist die Wand zumindest abschnittsweise aus einem porösen Material hergestellt, wobei das poröse Material aus der folgenden Gruppe ausgewählt sein kann: Zellulose, Nitrozellulose, Nylon, Agarose, Papier oder Pappe. Der Boden ist zweckmäßigerweise aus einem transparenten oder durchsichtigen Material hergestellt. Das erlaubt eine Messung der Absorption eines Laserstrahls im Durchlichtverfahren. Nach einem weiteren Ausgestaltungsmerkmal sind dritte oder vierte Moleküle mit der Wand oder den zweiten Molekülen assoziiert.

Weiterhin betrifft die Erfindung die Verwendung von Kapillaren, an deren Wänden die zweiten Moleküle immobilisiert sind, zur Durchführung des erfindungsgemäßen Verfahrens. Dabei können sich die Kapillaren, zumindest teilweise, durch unterschiedliche zweite Moleküle unterscheiden. Vorzugsweise unterscheiden sich die Kapillaren mit unterschiedlichen zweiten Molekülen zusätzlich, insbesondere durch deren Abmessungen oder Markierungen. Die Markierungen können aus weiteren Molekülen oder, insbesondere fluoreszierenden, Farbstoffen bestehen. Dadurch ist es möglich, Kapillaren zu identifizieren, an deren Wänden bestimmte zweite Moleküle immobilisiert sind. Es ist ferner möglich, daß die dritten oder vierten Moleküle mit der Wand assoziiert sind.

Nachfolgend wird die Erfindung durch die Zeichnung und anhand von Ausführungsbeispielen näher erläutert. Es zeigen
- Fig. 1a, 1b: eine schematische Darstellung des Durchstrahlens eines eine Lösung enthaltenen Gefäßes mit ersten Molekülen und immobilisierten zweiten Molekülen,
- Fig. 2a, 2b, 2c: eine schematische Darstellung des Durchstrahlens eines eine Lösung enthaltenen Gefäßes mit zweiten, vierten und ersten oder fünften Molekülen,
- Fig. 3a, 3b: eine schematische Darstellung des Durchstrahlens eines eine Lösung enthaltenen Gefäßes mit ersten, zweiten und dritten Molekülen,
- Fig. 4: ein Diagramm des Zeitverlaufs der in Vertiefungen eines 8-Well-Streifens detektierten Fluoreszenz Fluoreszenz-markierter Oligonukleotide,
- Fig. 5a, b, c: eine schematische Darstellung eines eine Lösung enthaltenden Gefäßes mit ersten Molekülen und immobilisierten zweiten Molekülen und
- Fig. 6a, b, c: eine schematische Darstellung eines eine Lösung enthaltenden Gefäßes mit ersten Molekülen, immobilisierten zweiten Molekülen und dritten Molekülen.

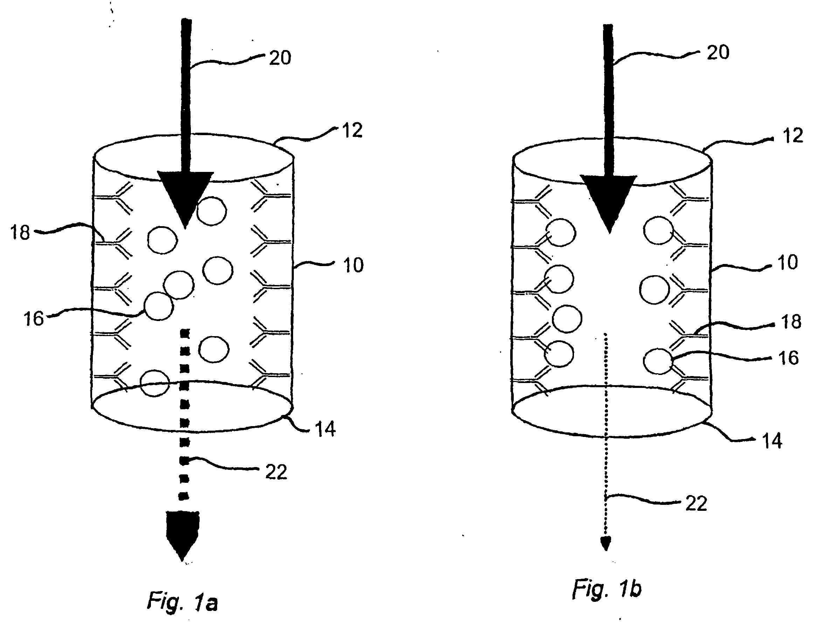

Fig. 1a zeigt einen Ausschnitt aus einem Gefäß 10 mit einem ersten Ende 12 und einem zweiten Ende 14. In dem Gefäß 10 befindet sich eine Lösung, welche die ersten Moleküle 16 enthält. An der Wand des Gefäßes 10 sind die dazu affinen zweiten Moleküle 18 immobilisiert. Der in das Gefäß 10 an dem ersten Ende 12 eingestrahlte Lichtstrahl 20 wird durch eine Wechselwirkung mit den ersten Molekülen 16, beispielsweise durch Absorption, moduliert. Der am zweiten Ende 14 ausstrahlende Lichtstrahl 22 weist gegenüber dem eingestrahlten Lichtstrahl 20 veränderte Eigenschaften auf. Die Veränderung dieser Eigenschaften ist ein Maß für die Konzentration der ersten Moleküle 16 in der Lösung. Fig. 1b zeigt das in Fig. 1a dargestellte Gefäß 10 nach dem Inkubieren. Die ersten Moleküle 16 sind an die zweiten Moleküle 18 gebunden. Der in das Gefäß 10 eingestrahlte Lichtstrahl 20 wird nicht durch Wechselwirkungen mit den ersten Molekülen 16 moduliert. Im Falle lichtabsorbierender erster Moleküle 16 ist der ausstrahlende Lichtstrahl 22 nur durch die Lichtabsorption der Lösung, nicht aber wie in Fig. 1a durch die Lichtabsorption der ersten Moleküle 16 abgeschwächt.

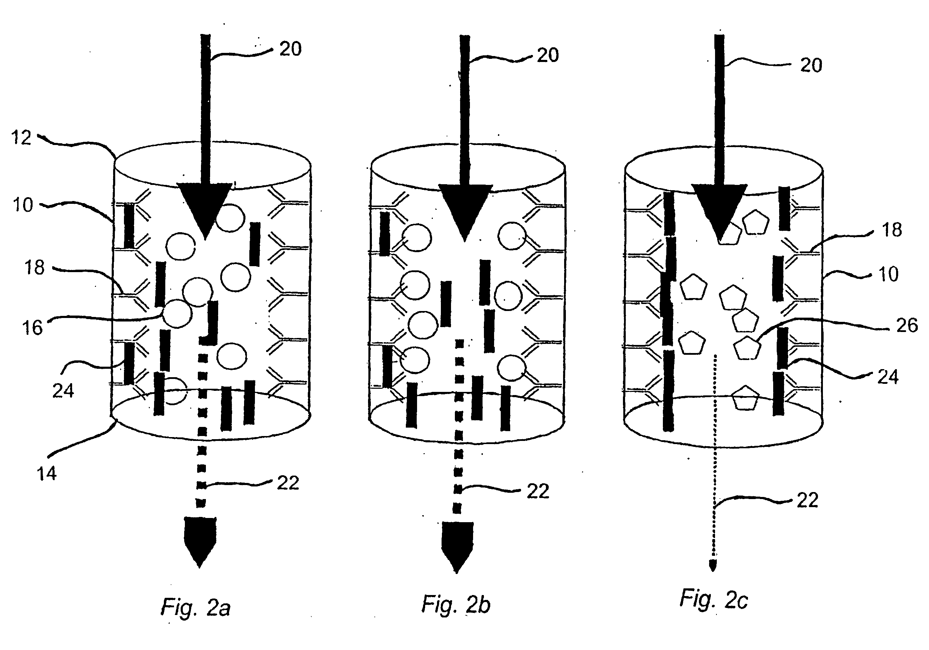

In Fig. 2a ist ein Ausschnitt aus einem Gefäß 10 mit daran gebundenen zweiten Molekülen 18 dargestellt. Die in dem Gefäß 10 enthaltene Lösung enthält erste Moleküle 16 und vierte Moleküle 24, die beide eine Affinität zu den zweiten Molekülen 18 aufweisen. Der eingestrahlte Lichtstrahl 20 wird hier durch eine Wechselwirkung mit den vierten Molekülen 24, nicht aber mit den ersten Molekülen 16 moduliert. Die Wechselwirkung kann dadurch bedingt sein, daß die vierten Moleküle 24 lichtabsorbierend sind. Der ausstrahlende Lichtstrahl 22 weist gegenüber dem einstrahlenden Lichtstrahl 20 veränderte Eigenschaften auf. Fig. 2b zeigt die Situation nach dem Inkubieren. Die ersten Moleküle 16 sind an die zweiten Moleküle 18 gebunden. Sie verhindern das Binden der vierten Moleküle 24 an die zweiten Moleküle 18. Die Konzentration der vierten Moleküle 24 in der Lösung ist unverändert. Die Eigenschaften des ausstrahlenden Lichtstrahls 22 sind gegenüber der in Fig. 2a dargestellten Situation nicht verändert. Das zeigt eine Bindung der ersten Moleküle 16 an die zweite Moleküle 18 an. Fig. 2c zeigt die Situation nach dem Inkubieren, wenn in der Lösung zuvor statt der ersten Moleküle 16 fünfte Moleküle 26 enthalten waren, die keine Affinität zu den zweiten Molekülen 18 aufweisen. Die vierten Moleküle 24 binden dann während des Inkubierens an die zweiten Moleküle 18. Nach dem Inkubieren wird der eingestrahlte Lichtstrahl 20 nicht durch eine Wechselwirkung mit den vierten Molekülen 24 moduliert. Im Falle lichtabsorbierender vierter Moleküle 24 ist die Intensität des ausstrahlenden Lichtstrahls 22 stärker als zu Beginn der Inkubation.

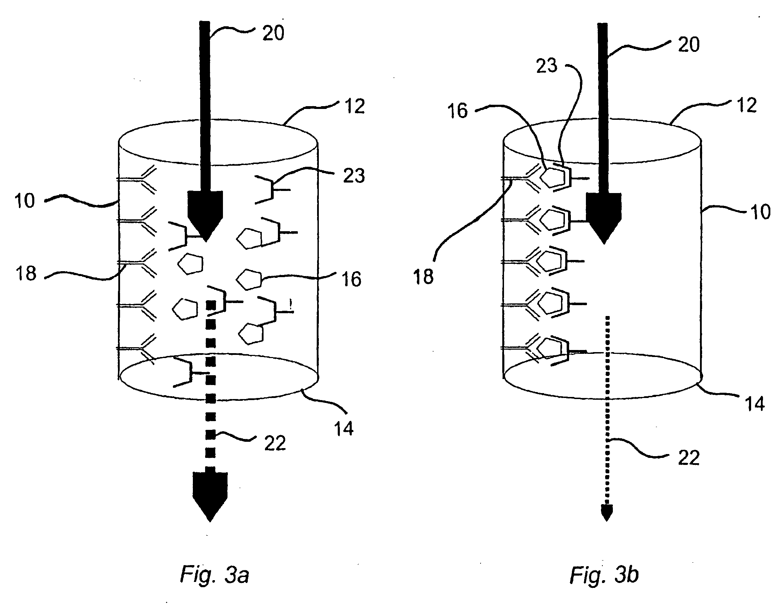

Fig. 3a zeigt einen Ausschnitt aus einem Gefäß 10. In dem Gefäß 10 ist eine Lösung mit ersten Molekülen 16 und dazu affinen dritten Molekülen 23 enthalten. An der Wand des Gefäßes 10 sind zweite Moleküle 18 immobilisiert. Sie weisen eine Affinität zu den ersten Molekülen 16 auf. Der in das Gefäß 10 an dem ersten Ende 12 eingestrahlte Lichtstrahl 20 wird durch eine Wechselwirkung mit den dritten Molekülen 23, beispielsweise durch Absorption, moduliert. Der ausstrahlende Lichtstrahl 22 weist gegenüber dem einstrahlenden Lichtstrahl 20 veränderte Eigenschaften auf. Fig. 3b zeigt die Situation nach dem Inkubieren. Die ersten Moleküle 16 sind an die zweiten Moleküle 18 und die dritten Moleküle 23 gebunden. Die Bindungsstellen der zweiten 18 und dritten Moleküle 23 an den ersten Molekülen 16 sind nicht identisch. Die dritten Moleküle 23 sind der Lösung entzogen. Der in das Gefäß 10 eingestrahlte Lichtstrahl 20 wird nicht mehr durch Wechselwirkungen mit den dritten Molekülen 23 moduliert.

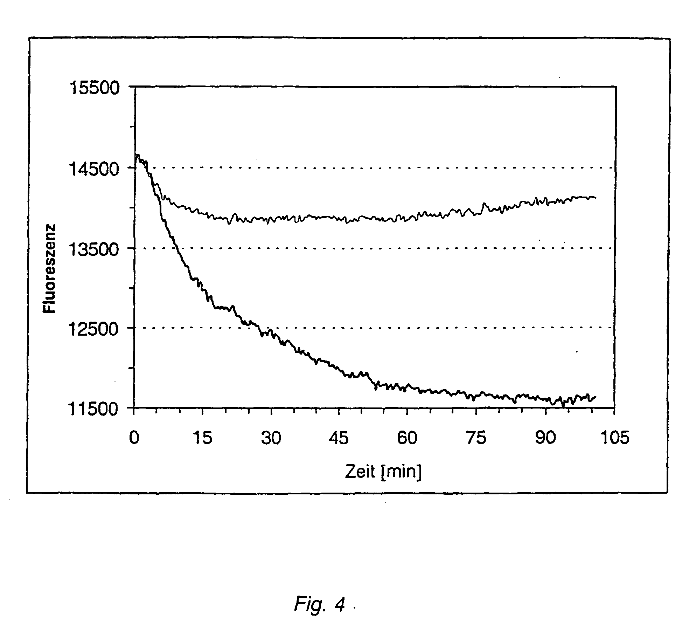

Fig. 4 zeigt den Zeitverlauf der in Streptavidin-beschichteten Vertiefungen eines 8-Well-Streifens (Boehringer Mannheim, Nr. 1664778) detektierten Fluoreszenz Fluoreszenz-markierter Oligonukleotide I. Eine Vertiefung I des 8-Well-Streifens ist mit einem am 5'-Terminus biotinylierten Oligonukleotid II der Sequenz 5'-TAA CAC AAC TGG TGT GCT CCT GGA-3' gemäß Sequenzprotokoll Nr. 1 beschichtet worden. Dazu sind in die Vertiefung I 300 µl einer Puffer-Lösung I mit 167 nM des Oligonukleotids II eingefüllt worden. Die Puffer-Lösung I besteht aus einer wässerigen Lösung von 10 mM TrisCL und 1 mM EDTA, pH 8,0. Eine Vertiefung II des 8-Well-Streifens ist nur mit 300 µl Puffer-Lösung I gefüllt worden. Nach einer Stunde Inkubation bei 37°C sind die Puffer-Lösungen I aus den Vertiefungen I und II abgesaugt worden. Die Vertiefungen I und II sind 5 mal mit 320 µl der Puffer-Lösung I gewaschen worden. Anschließend sind in die Vertiefungen I und II je 300 µl einer Puffer-Lösung II mit 167 nM des am 5'-Terminus mit 5[6]-Carboxytetramethylrhodamin Fluoreszenz-markierten Oligonukleotids I der Sequenz 5'-GAG CTA GGA CCT CTT CTG TCC AGG AGC ACA CCA GTT GTG TTA-3' gemäß Sequenzprotokoll Nr. 2 eingefüllt worden. Die Puffer-Lösung II besteht aus einer wässerigen Lösung von 150 mM NaCl, 10 mM TrisCl, 1 mM EDTA und 0,2 % Tween-20, pH 8,0. Bei einer Anregung mit 540 nm ist die Fluoreszenz in den Vertiefungen I und II bei 590 nm gemessen worden. Das Messen erfolgte in 25 Sekunden-Intervallen bei Raumtemperatur mittels eines Mikrotiterplattenphotometers. In Fig. 4 ist die Fluoreszenz in willkürlichen Einheiten dargestellt. Die dickere Linie zeigt die in Vertiefung I und die dünnere Linie die in Vertiefung II gemessene Fluoreszenz. Die Abnahme der Fluoreszenz in Vertiefung II zeigt die unspezifische Bindung des Oligonukleotids I an der Wand der Vertiefung II an. Die Differenz zwischen der Abnahme der Fluoreszenz in Vertiefung II und der Abnahme der Fluoreszenz in Vertiefung I zeigt die spezifische Bindung des Oligonukleotids I an das Oligonukleotid II an.

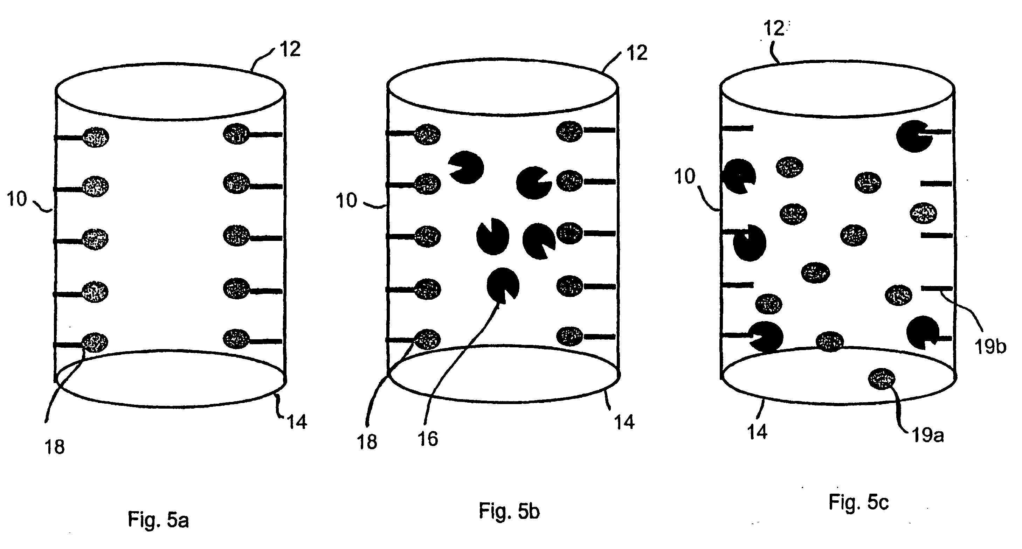

In den Fig. 5a bis c ist schematisch die Funktion eines Verfahrens zum Nachweisen und/oder Quantifizieren der katalytischen Aktivität erster Moleküle zu dazu affinen zweiten Molekülen gezeigt. Als erste Moleküle 16 werden hier Enzyme verwendet. An der Wand, nicht jedoch am Boden, des Gefäßes 10 sind zweite Moleküle 18 gebunden. Im vorliegenden Fall kann es sich dabei um Peptide, Nukleinsäuren, Zucker, Lipide und ähnliche Stoffe handeln, welche ein Substrat für das Enzym bilden. Die zweiten Moleküle 18 sind vorteilhafterweise mit einem Fluorophor markiert. Der Strahlengang des das Gefäß 10 durchstrahlenden Lichtstrahls (hier nicht gezeigt) verläuft parallel zu den Wänden. Er erfaßt nicht den Bereich der zweiten Moleküle 18.

Fig. 5b zeigt das Gefäß 10 nach Zugabe der nachzuweisenden ersten Moleküle 16. Als erste Moleküle 16 können hier beispielsweise Enzyme, wie Proteasen, Nukleasen, Zucker-abbauende Enzyme, Lipasen oder andere Enzyme verwendet werden. Die ersten Moleküle können auch Teile von größeren Enzymkomplexen oder Zellen sein. Weiterhin können die ersten Moleküle von Zellen gebildet und/oder sezerniert werden.

Fig. 5c zeigt das Gefäß 10 nach dem Inkubieren. Durch die Aktivität der als erste Moleküle 16 hier beispielsweise verwendeten Enzyme sind von den zweiten Molekülen 18 Fragmente 19a abgespalten worden. Reste 19b verbleiben an der Wand des Gefäßes 10, während die durch die Aktivität der ersten Moleküle 16 gebildeten Fragmente 19a sich gleichmäßig über das Volumen des Gefäßes 10 verteilen. Sie absorbieren Licht des eingestrahlten (hier nicht gezeigten) Lichtstrahls. Durch die Absorption des Lichts kann die Aktivität der ersten Moleküle 16 nachgewiesen werden.

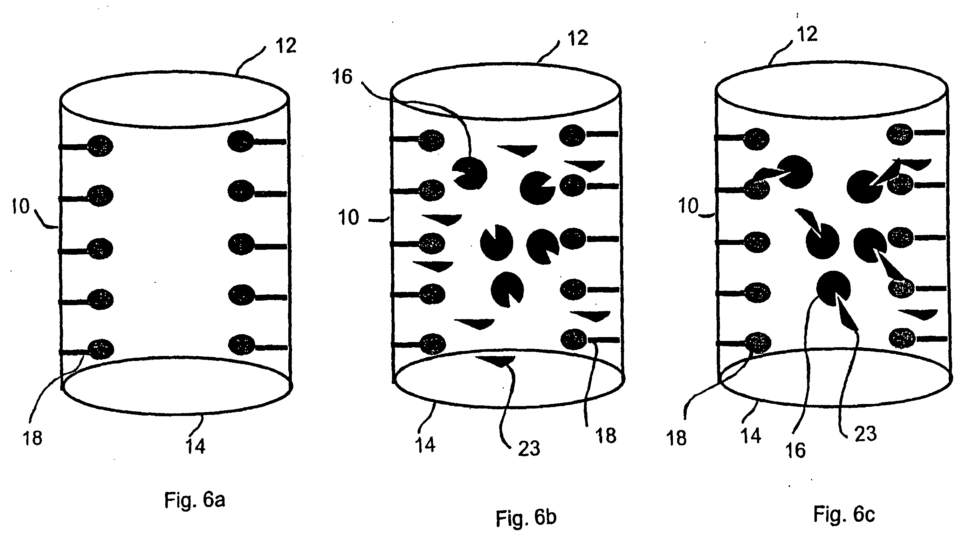

Die Fig. 6a bis c zeigen schematisch den Verfahrensablauf bei der Zugabe eines Enzyms als erstes Molekül 16 unter Verwendung eines Kompetitors als dritten Molekül 23. In einem Gefäß 10 sind an den Wänden, nicht jedoch am Boden, zweite Moleküle 18 immobilisiert. Bei den zweiten Molekülen 18 kann es sich beispielsweise um Peptide, Nukleinsäuren, Zucker, Lipide oder andere Stoffe handeln, welche ein Substrat für das Enzym bilden. Vorteilhafterweise sind die zweiten Moleküle 18 z.B. mit einem Fluorophor markiert. Eine (hier nicht gezeigter) Lichtstrahl durchdringt das Gefäß 10 parallel zu dessen Wänden, wobei der Bereich der zweiten Moleküle 18 nicht vom Licht beaufschlagt wird. Alternativ zum Lichtstrahl kann auch die Fluoreszenz in einem zentralen Bereich des Gefäßes 10 beobachtet werden.

Fig. 6b zeigt das Gefäß 10 nach Zugabe der ersten Moleküle 16. Im Gefäß befinden sich dritte Moleküle 23. Im vorliegenden Beispiel werden als erste Moleküle 16 Kompetitoren und als dritte Moleküle 23 Proteasen verwendet. Als dritte Moleküle 23 kommen aber auch Nukleasen, Zucker-abbauende Enzyme, Lipasen oder andere Enzyme in Betracht. Als erste Moleküle 16 kommen auch Inhibitoren, Enhancer, Induktoren und dgl. in Betracht.

Fig. 6c zeigt das Gefäß 10 nach dem Inkubieren der nachzuweisenden ersten Moleküle 16 mit den dritten Molekülen 23 sowie den immobilisierten zweiten Molekülen 18. Durch die Aktivität der als erste Moleküle 16 benutzten Kompetitoren ist die Aktivität der dritten Moleküle 23, hier Proteasen, spezifisch verändert worden. Sofern es sich bei den ersten Molekülen 16 um Inhibitoren der dritten Moleküle 23 handelt, wird der Abbau der zweiten Moleküle 18 spezifisch inhibiert. Die Inhibierung kann anhand der im Gefäß 10 gebildeten Fragmente 19a detektiert werden. Eine verringerte Zunahme an gebildeten Fragmenten 19a im Vergleich zu einer Kontrolle ohne Zugabe erster Moleküle 16 zeigt die Anwesenheit der ersten Moleküle 16 in dem Gefäß 10 an.

### Bezugszeichenliste

- 10: Gefäß,
- 12: erstes Ende,
- 14: zweites Ende,
- 16: erste Moleküle,
- 18: zweite Moleküle,
- 19a: Fragment,
- 19b: Rest,
- 20: eingestrahlter Lichtstrahl,
- 22: ausstrahlender Lichtstrahl,
- 23: dritte Moleküle,
- 24: vierte Moleküle,
- 26: fünfte Moleküle

## Patentansprüche

1. Verfahren zum Nachweisen und/oder Quantifizieren des Bindens erster Moleküle (16) an dazu affine zweite Moleküle (18) mit folgenden Schritten:
a) Vorsehen eines Gefäßes (10), in dem die zweiten Moleküle (18) an einer Wand immobilisiert sind,
b) Inkontaktbringen der zweiten Moleküle (18) mit einer die ersten Moleküle (16) enthaltenden Lösung,
c) Inkubieren des Gefäßes (10), so daß die ersten Moleküle (16) an die zweiten Moleküle (18) binden und sich an der Wand anreichern und
d) Detektieren der Konzentrationsänderung der ersten Moleküle (16) in der im Gefäß (10) enthaltenen Lösung mittels Strahlung, ohne Lösung aus dem Gefäß zu entnehmen.

2. Verfahren zum Nachweisen und/oder Quantifizieren des Bindens erster Moleküle (16) an dazu affine zweite Moleküle (18) mit folgenden Schritten:
a) Vorsehen eines Gefäßes (10), in dem die zweiten Moleküle (18) an einer Wand immobilisiert sind,
b) Inkontaktbringen der zweiten Moleküle (18) mit einer die ersten Moleküle (16) enthaltenden Lösung, wobei dritte Moleküle (23) mit einer Affinität zu den ersten Molekülen (16) in der Lösung oder in dem Gefäß (10) enthalten sind,
c) Inkubieren des Gefäßes (10), so daß die ersten Moleküle (16) an die zweiten (18) und dritten Moleküle (23) binden und sich an der Wand anreichern und
d) Detektieren der Konzentrationsänderung der dritten Moleküle (23) in der im Gefäß (10) enthaltenen Lösung mittels Strahlung, ohne Lösung aus dem Gefäß zu entnehmen.

3. Verfahren zum Nachweisen und/oder Quantifizieren des Bindens erster Moleküle (16) an dazu affine zweite Moleküle (18) mit folgenden Schritten:
a) Vorsehen eines Gefäßes (10), in dem die zweiten Moleküle (18) an einer Wand immobilisiert sind,
b) Inkontaktbringen der zweiten Moleküle (18) mit einer die ersten Moleküle (16) enthaltenden Lösung, wobei vierte Moleküle (24) mit einer Affinität zu den zweiten Molekülen (18) in der Lösung oder in dem Gefäß (10) enthalten sind,
c) Inkubieren des Gefäßes (10), so daß die ersten Moleküle (16) an die zweiten Moleküle (18) binden und die vierten Moleküle (24), zumindest teilweise, in Lösung gehen oder bleiben und
d) Detektieren der Konzentrationsänderung der vierten Moleküle (24) in der im Gefäß (10) enthaltenen Lösung mittels Strahlung, ohne Lösung aus dem Gefäß zu entnehmen.

4. Verfahren zum Nachweisen und/oder Quantifizieren der enzymatischen oder chemischen Aktivität erster Moleküle (16) zu dazu affinen zweiten Molekülen (18) mit folgenden Schritten:
a) Vorsehen eines Gefäßes (10), in dem die zweiten Moleküle (18) an einer Wand immobilisiert sind,
b) Inkontaktbringen der zweiten Moleküle (18) mit einer die ersten Moleküle (16) enthaltenden Lösung,
c) Inkubieren des Gefäßes (10), so daß die ersten Moleküle (16) die zweiten Moleküle (18) unter Freisetzung eines Fragments (19a) der zweiten Moleküle (18) umsetzen und
d) Detektieren der Konzentrationsänderung des Fragments (19a) in der im Gefäß (10) enthaltenen Lösung mittels Strahlung, ohne Lösung aus dem Gefäß zu entnehmen.

5. Verfahren zum Nachweisen und/oder Quantifizieren der enzymatischen oder chemischen Aktivität erster Moleküle (16) zu dazu affinen zweiten (18) oder dritten Molekülen mit folgenden Schritten:
a) Vorsehen eines Gefäßes (10), in dem die zweiten Moleküle (18) an einer Wand immobilisiert sind,
b) Inkontaktbringen der zweiten Moleküle (18) mit einer die ersten Moleküle (16) enthaltenden Lösung, wobei in der Lösung dritte Moleküle mit einer Affinität zu den zweiten Molekülen enthalten sind,
c) Inkubieren des Gefäßes (10), so daß die ersten Moleküle (16) an die zweiten (18) oder dritten Moleküle binden und dadurch eine die Freisetzung eines Fragments (19a) der zweiten Moleküle (18) bewirkende Umsetzung durch die dritten Moleküle unterdrückt oder verstärkt wird und
d) Detektieren der Konzentrationsänderung des Fragments (19a) in der im Gefäß (10) enthaltenen Lösung mittels Strahlung, ohne Lösung aus dem Gefäß (10) zu entnehmen.

6. Verfahren nach Anspruch 2 oder 3, wobei die in dem Gefäß (10) enthaltenen dritten (23) oder vierten Moleküle (24) mit den zweiten Molekülen (18) oder der Wand assoziiert sind.

7. Verfahren nach Anspruch 3, wobei die Affinität der vierten Moleküle (24) zu den zweiten Molekülen (18) nicht oder nicht wesentlich größer ist als die Affinität der ersten Moleküle (16) zu den zweiten Molekülen (18).

8. Verfahren nach Anspruch 3, wobei die Anzahl der ersten Moleküle (16) größer ist als die Anzahl der zweiten Moleküle (18) und die Anzahl der zweiten Moleküle (18) größer ist als die Anzahl der vierten Moleküle (24).

9. Verfahren nach Anspruch 1 oder 2, wobei die Anzahl der zweiten Moleküle (18) größer ist als die Anzahl der ersten Moleküle (16).

10. Verfahren nach Anspruch 2, wobei die Anzahl der ersten Moleküle (16) nicht kleiner ist als die Anzahl der dritten Moleküle (23).

11. Verfahren nach Anspruch 4, wobei das erste Molekül (16) ein spaltendes Enzym, vorzugsweise eine Protease, Peptidase, Nuklease, Helicase oder Lipase, oder ein Zucker-abbauendes Enzym ist.

12. Verfahren nach Anspruch 4 oder 11, wobei das zweite Molekül (18) ein, vorzugsweise aus einem Protein, Peptid, einer Nukleinsäure, Helicase oder einem monomeren oder polymeren Zucker gebildetes, Substrat für das erste Molekül (16) bildet.

13. Verfahren nach Anspruch 5, wobei das dritte Molekül ein spaltendes Enzym, vorzugsweise eine Protease, Peptidase, Nuklease, Helicase oder Lipase, oder ein Zucker-abbauendes Enzym ist.

14. Verfahren nach Anspruch 5, wobei das zweite Molekül (18) ein, vorzugsweise aus einem Protein, Peptid, einer Nukleinsäure, Helicase oder einem monomeren oder polymeren Zucker gebildetes, Substrat für das dritte Molekül bildet.

15. Verfahren nach Anspruch 5, wobei das erste Molekül (16) in bezug zum dritten Molekül ein Agonist, ein Antagonist oder ein Kompetitor ist.

16. Verfahren nach Anspruch 5, wobei durch die Bindung des zweiten Moleküls an das erste Molekül eine Spaltung des zweiten Moleküls durch das dritte Molekül verhindert wird.

17. Verfahren nach einem der vorhergehenden Ansprüche, wobei zum Detektieren mit einem Lichtstrahl (20), insbesondere einer definierten Wellenlänge, vorzugsweise einem Laserstrahl, in die Lösung eingestrahlt wird.

18. Verfahren nach Anspruch 17, wobei der Lichtstrahl (20) parallel zu der Wand eingestrahlt wird.

19. Verfahren nach Anspruch 17 oder 18, wobei der Lichtstrahl (20) polarisiert ist.

20. Verfahren nach einem der vorhergehenden Ansprüche, wobei zum Detektieren eine Fluoreszenz, eine Streuung, eine Absorption oder eine optische Aktivität gemessen wird.

21. Verfahren nach einem der vorhergehenden Ansprüche, wobei in der Lösung zusätzlich fünfte, als interne Marker dienende Moleküle (26) enthalten sind, die keine spezifische Affinität zu den ersten (16), zweiten (18), dritten (23) oder vierten Molekülen (24) aufweisen.

22. Verfahren nach einem der vorhergehenden Ansprüche, wobei die ersten (16), dritten (23), vierten (24) und/oder fünften Moleküle (26) oder Fragmente dieser Moleküle oder damit assoziierende Bestandteile fluoreszierend, lichtstreuend, lichtabsorbierend oder optisch aktiv sind.

23. Verfahren nach einem der vorhergehenden Ansprüche, wobei die Durchführung des Schritts lit. d durch mehrfaches oder kontinuierliches Bestimmen der Konzentration der ersten (16), dritten (23) oder vierten Moleküle (24) während der Durchführung des Schritts lit. c, insbesondere an deren Beginn und Ende, erfolgt.

24. Verfahren nach einem der vorhergehenden Ansprüche, wobei freie Bindungsstellen an der Wand des Gefäßes (10) durch daran gebundene sechste Moleküle abgesättigt sind.

25. Verfahren nach einem der vorhergehenden Ansprüche, wobei die Lösung mindestens einen eine unspezifische Bindung inhibierenden Zusatz, insbesondere ein Detergenz, ein Protein, ein Proteingemisch oder ein Salz, enthält.

26. Verfahren nach einem der vorhergehenden Ansprüche, wobei zur Ermittlung einer spezifischen Konzentrationsänderung von dem Betrag der Konzentrationsänderung gemäß Schritt lit. d der Betrag einer Konzentrationsänderung bei Durchführung des Verfahrens ohne zweite Moleküle abgezogen wird.

27. Verfahren nach einem der vorhergehenden Ansprüche, wobei das Gefäß (10) als Kavität einer Mikrotiterplatte mit mindestens 96, insbesondere 384, Kavitäten ausgebildet ist.

28. Verfahren nach einem der vorhergehenden Ansprüche, wobei das Gefäß (10) an der Ein- und/oder Austrittsstelle des Lichtstrahls (20, 22), vorzugsweise dem Boden des Gefäßes (10), im wesentlichen keine immobilisierten zweiten Moleküle (18) aufweist.

29. Verfahren nach einem der vorhergehenden Ansprüche, wobei das Gefäß (10) eine an den Enden (12, 14) offene Kapillare ist.

30. Verfahren nach Anspruch 29, wobei die Kapillare mittels Kapillarkräften mit der Lösung gefüllt wird.

31. Verfahren nach einem der vorhergehenden Ansprüche, wobei der Quotient aus der Fläche der Wand in mm² und dem Volumen der Lösung in mm³ größer als 1 mm⁻¹, vorzugsweise größer als 3 mm⁻¹, ist.

32. Verfahren nach einem der vorhergehenden Ansprüche, wobei die ersten (16), zweiten (18), dritten (23), vierten (24), fünften (26) oder sechsten Moleküle aus folgender Gruppe ausgewählt sind: Peptide, Proteine, Nukleinsäuren, Zucker, Polymere, Botenstoffe, Zellen, Zellfragmente, Viren, deren Bestandteile oder Fragmente dieser Bestandteile, Kapside, deren Bestandteile oder Fragmente dieser Bestandteile und Hormone.

33. Verfahren nach einem der vorhergehenden Ansprüche, wobei das Verfahren gleichzeitig oder in kurzer Abfolge an einer Anzahl von Gefäßen (10), insbesondere Kapillaren, durchgeführt wird.

34. Mikrotiterplatte zur Durchführung eines Verfahrens nach einem der Ansprüche 1 - 33 mit einer Vielzahl jeweils eine Wand und einen Boden aufweisenden Kavitäten, wobei die Wand für die Bindung zweiter Moleküle (18) aktiviert ist,
**dadurch gekennzeichnet, daß**
der Boden nicht für die Bindung zweiter Moleküle (18) aktiviert ist.

35. Mikrotiterplatte nach Anspruch 34, wobei an der Wand zweite Moleküle (18) immobilisiert sind und der Boden im wesentlichen keine immobilisieren zweiten Moleküle (18) aufweist.

36. Mikrotiterplatte nach Anspruch 34 oder 35, wobei die Wand zumindest abschnittsweise aus einem porösen Material hergestellt ist.

37. Mikrotiterplatte nach Anspruch 36, wobei das poröse Material aus der folgenden Gruppe ausgewählt ist: Zellulose, Nitrozellulose, Nylon, Agarose, Papier, Pappe.

38. Mikrotiterplatte nach einem der Ansprüche 34 bis 37, wobei der Boden aus einem transparenten oder durchsichtigen Material hergestellt ist.

39. Mikrotiterplatte nach Anspruch 34 bis 38, wobei dritte (23) oder vierte Moleküle (24) mit der Wand oder den zweiten Molekülen (18) assoziiert sind.

40. Verwendung von Kapillaren, an deren Wand zweite Moleküle (18) immobilisiert sind, zur Durchführung des Verfahrens nach einem der Ansprüche 1 - 33.

41. Verwendung nach Anspruch 40, wobei sich die Kapillaren eine die Art der zweiten Moleküle (18) anzeigende Markierung aufweisen.

42. Verwendung nach Anspruch 40 oder 41, wobei die dritten (23) oder vierten Moleküle (24) mit der Wand oder den zweiten Molekülen (18) assoziiert sind.

## Claims

1. Method for detecting and/or quantifying of the binding of first molecules (16) to thereto affinitive second molecules (18), consisting of the following steps:
a) provision of a vessel (10) in which the second molecules (18) are immobilised on a wall,
b) bringing the second molecules (18) into contact with a solution containing the first molecules (16),
c) incubating the vessel (10) so that the first molecules (16) bind to the second molecules (18) and concentrate themselves on the wall and
d) detecting the change in concentration of the first molecules (16) in the solution contained in the vessel (10) via radiation without removing solution from the vessel.

2. Method for detecting and/or quantifying of the binding of first molecules (16) to thereto affinitive second molecules (18), consisting of the following steps:
a) provision of a vessel (10) in which the second molecules (18) are immobilised on a wall,
b) bringing the second molecules (18) into contact with a solution containing the first molecules (16), wherein third molecules (23) with an affinity to the first molecules (16) are contained in the solution or in the vessel (10),
c) incubating the vessel (10) so that the first molecules (16) bind to the second (18) and third molecules (23) and concentrate themselves on the wall and
d) detecting the change in concentration of the third molecules (23) in the solution contained in the vessel (10) via radiation without removing any solution from the vessel.

3. Method for detecting and/or quantifying of the binding of first molecules (16) to thereto affinitive second molecules (18), consisting of the following steps:
a) provision of a vessel (10) in which the second molecules (18) are immobilised on a wall,
b) bringing the second molecules (18) into contact with a solution containing the first molecules (16), wherein fourth molecules (24) with an affinity to the second molecules (18) are contained in the solution or in the vessel (10),
c) incubating the vessel (10) so that the first molecules (16) bind to the second molecules (18) and the fourth molecules (24), at least partially, enter the solution or remain in the solution and
d) detecting the change in concentration of the fourth molecules (24) in the solution contained in the vessel (10) via radiation without removing any solution from the vessel.

4. Method for detecting and/or quantifying of the enzymic or chemical activity of first molecules (16) towards thereto affinitive second molecules (18), consisting of the following steps:
a) provision of a vessel (10) in which the second molecules (18) are immobilised on a wall,
b) bringing the second molecules (18) into contact with a solution containing the first molecules (16),
c) incubating the vessel (10) so that the first molecules (16) convert the second molecules (18) which conversion releases a fragment (19a) of the second molecules (18) and
d) detecting the change in concentration of the fragment (19a) in the solution contained in the vessel (10) via radiation without removing any solution from the vessel.

5. Method for detecting and/or quantifying of the enzymic or chemical activity of first molecules (16) towards thereto affinitive second (18) or third molecules, consisting of the following steps:
a) provision of a vessel (10) in which the second molecules (18) are immobilised on a wall,
b) bringing the second molecules (18) into contact with a solution containing the first molecules (16), wherein third molecules with an affinity to the second molecules are contained in the solution,
c) incubating the vessel (10) so that the first molecules (16) bind to the second (18) or third molecules and are suppressed or intensified due to a conversion due to the third molecules which causes the release of a fragment (19a) of the second molecules (18), and
d) detecting the change in concentration of the fragment (19a) in the solution contained in the vessel (10) via radiation without removing any solution from the vessel (10).

6. Method as defined in claim 2 or 3, wherein the third (23) or fourth (24) molecules contained in the vessel (10) are associated with the second molecules (18) or the wall.

7. Method as defined in claim 3, wherein the affinity of the fourth molecules (24) to the second molecules (18) is not greater or is not significantly greater than the affinity of the first molecules (16) to the second molecules (18).

8. Method as defined in claim 3, wherein the number of first molecules (16) is greater than the number of second molecules (18) and the number of second molecules (18) is greater than the number of fourth molecules (24).

9. Method as defined in claim 1 or 2, wherein the number of second molecules (18) is greater than the number of first molecules (16).

10. Method as defined in claim 2, wherein the number of first molecules (16) is not less than the number of third molecules (23).

11. Method as defined in claim 4, wherein the first molecule (16) is a cleaving enzyme, preferably a protease, peptidase, nuclease, helicase or lipase or an enzyme which breaks down sugar.

12. Method as defined in claim 4 or 11, wherein the second molecule (18) creates for the first molecule (16) a substrate , preferably from a protein, a peptide, a nucleic acid, helicase or a monomer or polymer sugar.

13. Method as defined in claim 5, wherein the third molecule is a cleaving enzyme, preferably a protease, peptidase, nucleic acid, helicase or lipase or an enzyme which breaks down sugar.

14. Method as defined in claim 5, wherein the second molecule (18) creates for the third molecule a substrate preferably of a protein, peptide, a nucleic acid, helicase or a monomer or polymer sugar.

15. Method as defined in claim 5, wherein the first molecule (16) is an agonist, an antagonist or a competitor in relation to the third molecule.

16. Method as defined in claim 5, wherein a cleavage of the second molecule by the third molecule is prevent by the binding of the second molecule to the first molecule.

17. Method as defined in one of the preceding claims, wherein, for detecting, a beam of light (20), particularly with a defined wave length, preferably a laser beam, is directed into the solution.

18. Method as defined in claim 17, wherein the beam of light (20) is directed parallel to the wall.

19. Method as defined in claim 17 or 18, wherein the beam of light (20) is polarised.

20. Method as defined in one of the preceding claims, wherein, for detecting, a fluorescence, a diffusion, an absorption or an optical activity is measured.

21. Method as defined in one of the preceding claims, wherein the solution also contains fifth molecules (26) which are used as internal markers, and which have no specific affinity to the first (16), second (18), third (23) or fourth (24) molecules.

22. Method as defined in one of the preceding claims, wherein the first (16), third (23), fourth (24) and/or fifth (26) molecules or fragments of these molecules or herewith associating components are fluorescing, light-diffusing, light-absorbing or optically active.

23. Method as defined in one of the preceding claims, wherein the execution of the step d occurs through multiple or continuous determination of the concentration of the first (16), third (23) or fourth (24) molecules during the execution of step c, particularly at their beginning and end.

24. Method as defined in one of the preceding claims, wherein free binding sites on the wall of the vessel (10) are saturated by sixth molecules which are bonded thereto.

25. Method as defined in one of the preceding claims, wherein the solution contains at least one addition which inhibits a non-specific bond, in particular a detergent, a protein, a protein mixture or a salt.

26. Method as defined in one of the preceding claims, wherein, to determine a specific change of concentration , the amount of concentration change during execution of the method without second molecules is deducted from the amount of concentration change in accordance with step d.

27. Method as defined in one of the preceding claims, wherein the vessel (10) is formed as cavity of a micro-titre plate with at least 96, in particular 384, cavities.

28. Method as defined in one of the preceding claims, wherein the vessel (10) has essentially no immobilised second molecules (18) on the place of entry and/or exit of the beam of light (20, 22), preferably on the floor of the vessel (10).

29. Method as defined in one of the preceding claims, wherein the vessel (10) is an open capillary at the ends (12, 14).

30. Method as defined in claim 29, wherein the capillary is filled with the solution via capillary action.

31. Method as defined in one of the preceding claims, wherein the quotient of the surface of the wall in mm² and the volume of the solution in mm³ is greater than 1 mm⁻¹, preferably greater than 3 mm⁻¹.

32. Method as defined in one of the preceding claims, wherein the first (16), second (18), third (23), fourth (24), fifth (26) or sixth molecules are selected from following group: peptides, proteins, nucleic acids, sugar, polymers, messenger substances, cells, cell fragments, viruses, their components or fragments of these components, capsules, their components or fragments of these components and hormones.

33. Method as defined in one of the preceding claims, wherein the method is executed simultaneously or in quick succession on a number of vessels (10), in particular capillaries.

34. Micro-titre plate for execution of a method as defined in one of the claims 1 to 33 with a plurality of cavities each having a wall and a floor, wherein the wall is activated for the binding of second molecules (18),
**characterised in that**
the floor is not activated for the binding of second molecules (18).

35. Micro-titre plate as defined in claim 34, wherein second molecules (18) are immobilised on the wall and the floor exhibits essentially no immobilised second molecules (18).

36. Micro-titre plate as defined in claim 34 or 35, wherein the wall or at least sections of it are made of a porous material.

37. Micro-titre plate as defined in claim 36, wherein the porous material is selected from the following group: cellulose, nitrocellulose, nylon, agarose, paper, cardboard.

38. Micro-titre plate as defined in one of the claims 34 to 37, wherein the floor is made of a transparent or clear material.

39. Micro-titre plate as defined in claim 34 to 38, wherein third (23) or fourth (24) molecules are associated with the wall or the second molecules (18).

40. Use of capillaries, on whose wall second molecules (18) are immobilised, for the execution of the method as defined in one of the claims 1 to 33.

41. Use as defined in claim 40, wherein the capillaries have a marking indicating the type of second molecules (18).

42. Use as defined in claim 40 or 41, wherein the third (23) or fourth (24) molecules are associated with the wall or the second molecules (18).

## Revendications

1. Procédé de détection et/ou de quantification de la liaison de premières molécules (16) à des deuxièmes molécules affines (18) selon les opérations suivantes:
a) prévoir un récipient (10) dans lequel les deuxièmes molécules (18) sont immobilisées sur une paroi,
b) mise en contact des deuxièmes molécules (18) avec une solution qui contient les premières molécules (16),
c) incubation du récipient (10) de façon que les premières molécules (16) lient avec les deuxièmes molécules (18) et s'enrichissent à la paroi,
d) détection de la modification de concentration des premières molécules (16) dans la solution contenue dans le récipient (10) à l'aide d'une radiation, sans prélever de solution du récipient.

2. Procédé de détection et/ou de quantification de la liaison des premières molécules (16) aux deuxièmes molécules affines (18) selon les opérations suivantes:
a) prévoir un récipient (10) dans lequel les deuxièmes molécules (18) sont immobilisées sur une paroi,
b) mise en contact des deuxièmes molécules (18) avec une solution qui contient les premières molécules (16), les troisièmes molécules (23) ayant une affinité avec les premières molécules (16) sont contenues dans la solution ou dans le récipient (10),
c) incubation du récipient (10) de façon que les premières molécules (16) lient avec les deuxièmes (18) et les troisièmes molécules (23) et s'enrichissent à la paroi,
d) détection de la modification de concentration des troisièmes molécules (23) dans la solution contenue dans le récipient (10) à l'aide d'une radiation, sans prélever de solution du récipient.

3. Procédé de détection et/ou de quantification de la liaison des premières molécules (16) aux deuxièmes molécules affines (18) selon les opérations suivantes:
a) prévoir un récipient (10) dans lequel les deuxièmes molécules (18) sont immobilisées sur une paroi,
b) mise en contact des deuxièmes molécules (18) avec une solution qui contient les premières molécules (16), les quatrièmes molécules (24) ayant une affinité avec les deuxièmes molécules (18) sont contenues dans la solution ou dans le récipient (10),
c) incubation du récipient (10) de façon que les premières molécules (16) lient avec les deuxièmes molécules (18) et les quatrièmes molécules (24), tout au moins partiellement, se dissolvent ou restent,
d) détection de la modification de concentration des quatrièmes molécules (24) dans la solution contenue dans le récipient (10) à l'aide d'une radiation, sans prélever de solution du récipient.

4. Procédé de détection et/ou de quantification de l'activité enzymatique ou chimique des premières molécules (16) avec les deuxièmes molécules (18) affines selon les opérations suivantes:
a) prévoir un récipient (10) dans lequel les deuxièmes molécules (18) sont immobilisées sur une paroi,
b) mise en contact des deuxièmes molécules (18) avec une solution qui contient les premières molécules (16),
c) incubation du récipient (10) de façon que les premières molécules (16) transforment les deuxièmes molécules (18) en libérant un fragment (19a) des deuxièmes molécules (18),
d) détection de la modification de concentration du fragment (19a) dans la solution contenue dans le récipient (10) à l'aide d'une radiation, sans prélever de solution du récipient.

5. Procédé de détection et/ou de quantification de l'activité enzymatique ou chimique des premières molécules (16) avec les deuxièmes (18) ou les troisièmes molécules affines selon les opérations suivantes:
a) prévoir un récipient (10) dans lequel les deuxièmes molécules (18) sont immobilisées sur une paroi,
b) mise en contact des deuxièmes molécules (18) avec une solution qui contient les premières molécules (16), des troisièmes molécules ayant une affinité avec les deuxièmes molécules sont contenues dans la solution,
c) incubation du récipient (10) de façon que les premières molécules (16) lient avec les deuxièmes (18) ou les troisièmes molécules et que, par cette liaison, une transformation provoquant la libération d'un fragment (19a) des deuxièmes molécules (18) soit supprimée ou renforcée,
d) détection de la modification de concentration du fragment (19a) dans la solution contenue dans le récipient (10) à l'aide d'une radiation, sans prélever de solution du récipient (10).

6. Procédé selon la revendication 2 ou 3, les troisièmes (23) ou les quatrièmes molécules (24) contenues dans le récipient (10) étant associées aux deuxièmes molécules (18) ou à la paroi.

7. Procédé selon la revendication 3, l'affinité des quatrièmes molécules (24) avec les deuxièmes molécules (18) n'étant pas ou pas nettement supérieure à l'affinité des premières molécules (16) avec les deuxièmes molécules (18).

8. Procédé selon la revendication 3, le nombre des premières molécules (16) étant supérieur au nombre des deuxièmes molécules (18) et le nombre des deuxièmes molécules (18) étant supérieur au nombre des quatrièmes molécules (24).

9. Procédé selon la revendication 1 ou 2, le nombre des deuxièmes molécules (18) étant supérieur au nombre des premières molécules (16).

10. Procédé selon la revendication 2, le nombre des premières molécules (16) n'étant pas inférieur au nombre des troisièmes molécules (23).

11. Procédé selon la revendication 4, la première molécule (16) étant une enzyme de décomposition, de préférence une protéase, peptidase, nucléase, hélicase, lipase ou une enzyme décomposant le sucre.

12. Procédé selon la revendication 4 ou 11, la deuxième molécule (18) formant un substrat pour la première molécule (16), de préférence d'une protéine, peptide, acide nucléique, hélicase ou d'un sucre monomère ou polymère.

13. Procédé selon la revendication 5, la troisième molécule étant une enzyme de décomposition, de préférence une protéase, peptidase, nucléase, hélicase, lipase ou une enzyme décomposant le sucre.

14. Procédé selon la revendication 5, la deuxième molécule (18) formant un substrat pour la troisième molécule, de préférence d'une protéine, peptide, acide nucléique, hélicase ou d'un sucre monomère ou polymère.

15. Procédé selon la revendication 5, la première molécule (16) étant un agoniste, un antagoniste ou un compétiteur par rapport à la troisième molécule.

16. Procédé selon la revendication 5, une coupure de la deuxième molécule, par la liaison de la deuxième molécule à la première molécule, étant inhibée par la troisième molécule.

17. Procédé selon l'une des revendications précédentes, un faisceau lumineux (20) pour la détection, en particulier d'une longueur d'onde définie, de préférence un faisceau laser, étant irradié dans la solution.

18. Procédé selon la revendication 17, le faisceau lumineux (20) étant irradié parallèlement à la paroi.

19. Procédé selon la revendication 17 ou 18, le faisceau lumineux (20) étant polarisé.

20. Procédé selon l'une des revendications précédentes, une fluorescence, une diffusion, une absorption ou une activité optique étant mesurée pour la détection.

21. Procédé selon l'une des revendications précédentes, des cinquièmes molécules (26) servant de marqueur interne sont contenues dans la solution, ces cinquièmes molécules ne présentent aucune affinité spécifique avec les premières (16), les deuxièmes (18), les troisièmes (23) ou les quatrièmes molécules (24).

22. Procédé selon l'une des revendications précédentes, les premières (16), les troisièmes (23), les quatrièmes (24) et/ou les cinquièmes molécules (26) ou des fragments de ces molécules ou des composants s'y associant, étant fluorescents, diffusants, absorbants ou optiquement actifs.

23. Procédé selon l'une des revendications précédentes, l'opération mentionnée au point d) étant effectuée par une détermination multiple ou continue de la concentration des premières (16), troisièmes (23) ou quatrièmes molécules (24) pendant l'exécution de l'opération mentionnée au point c), en particulier à son début et à la fin.

24. Procédé selon l'une des revendications précédentes, des sites liants libres étant saturés à la paroi du récipient (10) par des sixièmes molécules qui y sont liées.

25. Procédé selon l'une des revendications précédentes, la solution contenant au moins un additif inhibant une liaison non spécifique, en particulier un détergent , une protéine, un mélange de protéine ou un sel.

26. Procédé selon l'une des revendications précédentes, la somme d'une modification de concentration lors de l'exécution du procédé sans deuxième molécule étant soustrait de la somme de la modification de concentration selon l'opération mentionnée au point d) pour le calcul d'une modification de concentration spécifique.

27. Procédé selon l'une des revendications précédentes, le récipient (10) est tel qu'il forme une cavité d'une plaque microtiter avec au moins 96, en particulier 384 cavités.

28. Procédé selon l'une des revendications précédentes, le récipient (10) ne présentant essentiellement pas de deuxième molécule immobilisée (18) au point d'entrée et/ou de sortie du faisceau lumineux (20, 22), de préférence sur le fond du récipient (10).

29. Procédé selon l'une des revendications précédentes, le récipient (10) étant un capillaire ouvert aux extrémités (12, 14).

30. Procédé selon la revendication 29, le capillaire étant rempli de solution par les forces de capillarité.

31. Procédé selon l'une des revendications précédentes, le quotient de la surface de la paroi en mm² et du volume de la solution en mm³ étant supérieur à 1 mm⁻¹, de préférence supérieur à 3 mm⁻¹.

32. Procédé selon l'une des revendications précédentes, les premières (16), deuxièmes (18), troisièmes (23), quatrièmes (24), cinquièmes (26) ou sixièmes molécules du groupe suivant étant sélectionnées : peptide, protéine, acides nucléiques, sucre, polymères, messagers, cellules, fragments cellulaires, virus, leurs composants ou fragments de ces composants, capsides, leurs composants ou fragments de ces composants et hormones.

33. Procédé selon une des revendications précédentes, le procédé étant effectué simultanément ou successivement sur un nombre de récipients (10), en particulier de capillaires.

34. Plaque microtiter pour l'exécution d'un procédé selon l'une des revendications 1 à 33 avec une multiplicité de cavités présentant respectivement une paroi et un fond, la paroi étant activée pour la liaison des deuxièmes molécules (18),
**caractérisé par le fait que**
le fond n'est pas activé pour la liaison des deuxièmes molécules (18).

35. Plaque microtiter selon la revendication 34, les deuxièmes molécules (18) étant immobilisées sur la paroi et le fond ne présentant essentiellement pas de deuxièmes molécules (18) immobilisées.

36. Plaque microtiter selon la revendication 34 ou 35, la paroi étant, au moins partiellement, en matériau poreux.

37. Plaque microtiter selon la revendication 36, le matériau poreux étant sélectionné parmi le groupe suivant: cellulose, nitrocellulose, nylon, agarose, papier, carton.

38. Plaque microtiter selon l'une des revendications 34 à 37, le fond étant en matériau transparent.

39. Plaque microtiter selon les revendications 34 à 38, les troisièmes (23) ou quatrièmes molécules (24) étant associées à la paroi ou aux deuxièmes molécules (18).

40. Utilisation de capillaires sur les parois desquels sont immobilisées des deuxièmes molécules (18) pour l'exécution du procédé selon l'une des revendications 1 à 33.

41. Utilisation selon la revendication 40, les capillaires présentant un marquage indiquant la nature des deuxièmes molécules (18).

42. Utilisation selon la revendication 40 ou 41, les troisièmes (23) ou les quatrièmes molécules (24) étant associées à la paroi ou aux deuxièmes molécules (18).


### SEQUENZ PROTOKOLL

<110> november Aktiengesellschaft Gesellschaft für Molekulare Medizin
<120> Verfahren zum Nachweisen und/oder Quantifizieren des Bindens erster Moleküle
<130> 411674GA
<140>
   <141>
<160> 2
<170> PatentIn Ver. 2.1
<210> 1
   <211> 24
   <212> DNA
   <213> Künstliche Sequenz
<220>
   <223> Beschreibung der künstlichen Sequenz: Willkürlich gewählte Sequenz
<400> 1
   taacacaact ggtgtgctcc tgga                                          24
<210> 2
   <211> 42
   <212> DNA
   <213> Künstliche Sequenz
<220>
   <223> Beschreibung der künstlichen Sequenz: Willkürlich gewählte Sequenz
<400> 2
   gagctaggac ctcttctgtc caggagcaca ccagttgtgt ta                      42